# EUROPEAN PATENT APPLICATION

(11) **EP 4 181 155 A1**
(43) Date of publication of application: **17.05.2023**
(21) Application number: 21208393.5
(22) Date of filing: 16.11.2021
(51) Int. Cl.: G16H 40/20

(54) **GENERATING INFORMATION INDICATIVE OF AN INTERACTION**

(71) Applicant: Koninklijke Philips N.V., 5656 AG Eindhoven (NL)
(72) Inventor: BERA, Deep, Eindhoven (NL); BULUT, Murtaza, Eindhoven (NL); SARTOR, Francesco, Eindhoven (NL); HUIJBREGTS, Laurentia Johanna, Eindhoven (NL); VAN LIESHOUT, Ron Martinus Laurentius, Eindhoven (NL); TIEMANN, Christian Andreas, Eindhoven (NL)
(74) Representative: Philips Intellectual Property & Standards

(57) **Abstract**

In an embodiment, a computer-implemented method (100) is described. The method comprises receiving (102) a request associated with a user for information about a client. The method further comprises, in response to the request, generating (104) information indicative of at least one interaction between at least one caregiver and a client context associated with the client based on interaction data obtained responsive to the at least one interaction. The generated information is customized to the user based on a user profile associated with the user.

## Description

### TECHNICAL FIELD OF THE INVENTION

The invention relates to a method, a non-transitory machine-readable medium and apparatus for generating information indicative of an interaction.

### BACKGROUND OF THE INVENTION

An intensive care unit (ICU) is a complex environment, where a multidisciplinary team of caregivers (e.g., nurses, residents, physicians, respiratory therapist, etc.) monitor and interpret patients and their data.

Due to personal differences each caregiver (due to their experience, education, preferences, physical state, mental state, etc.) uses and interprets the patient data differently. For example, while one caregiver may be more interested and hence interacting more with basic monitoring outputs, such as changes in the vital signs, another caregiver may be more interested with the medications given. Additionally, there might be differences in their preference of visualization of these data and their way of associating these data either with events or artifacts.

These differences may be dependent on multiple factors including caregiver-related (e.g., experience, workload, and cognitive load, etc.), the patient (e.g., stable, unstable), the software environment (e.g., the operating system, application, software version, user interface, display setting, permissions, memory and storage capacity of the tools that are being used, etc.) and the physical environment (e.g., over crowdedness, workflow (handover), location, air quality and temperature, etc.). As there may be inter-caregiver differences (i.e. between different caregivers), it can be expected there are intra-caregiver (i.e. same caregiver interacting with data at different moments) differences as well. Exploring and quantifying all interactions with the patient data can be used as additional source of clinical information.

ICUs provide care to the most severely ill patients, and therefore they possess different and unique characteristics than the other hospital units. The ICU teams work in physically and emotionally challenging environments, and they are low in temporal stability (i.e., staff changes during patient treatment).

There may be various constraints associated with working in an ICU. These constraints include physical constraints such as near-constant alarms, uneven lighting, poorly placed equipment, space limitation. These constraints further include emotional constraints due to deaths, persistent grieving and moral distress.

A typical ICU team may include physicians or intensivists, clinical pharmacists, respiratory therapists, physiotherapists, dieticians, bedside nurses, clinical psychologists, and caregivers-in-training. The intensivist, a physician with specialized training in critical care medicine, is the leader of the team and has ultimate responsibility for medical decision-making.

Each caregiver is unique in terms of skill, experience, knowledge and behavior, which results in different style of working and communication (e.g. jargon, details, timing, etc.). The diversity of the team can be helpful for care, but it can also lead to ineffective and conflicting interactions, which may affect the patient care negatively.

Moreover, lifespan of an ICU team is small (i.e., low temporal stability) as team members can change daily, but the tasks of the team are longer than the life of the team. For example, a single patient may spend 14 days or longer in an ICU, during which team composition, and team dynamics may evolve. This is an additional challenge in terms of patient care due to the need for information sharing, the handover process and use of electronic health records.

Two kinds of discussions that can happen include formal and informal discussions. The most clinically important discussions are the formal discussions (i.e., 'formal rounds'), which represent the foundation of ICU team collaborations and decision making. Formal discussions may be daily rounds of face-to-face meetings that are attended by most, if not all, caregivers involved with patient care. Informal discussion can take place between small subset of team members throughout the day.

In a formal meeting where an ICU patient is discussed (either on bedside, or in a different room), most, if not all, team members are present. However, due to ICU needs, such meetings are a difficult endeavor. Ideally everyone in the team will share knowledge in an open and detailed manner, which may help in minimizing knowledge or training related errors. However, such meetings may be frequently interrupted by phone calls and clinical emergencies, which disturb the flow and increases the meeting duration. Caregivers may compensate for these normal and anticipated interruptions by withholding information during the rounds to speed up the meeting.

Informal meetings may be needed when (formal) rounds are not done in a timely manner, family members are present (which may prevent caregivers from discussing negative or sensitive information) and/or caregivers are unsure/reserved and did not speak up during other meetings. Informal discussions cannot be avoided, but they may be problematic in nature because they cause information gaps among care providers within the team.

During interaction with a patient or a context associated with the patient (e.g., a medical device associated with the patient), various data may be acquired related to the monitoring of physiological patient parameters. However, various issues exist during these interactions

There may be times when false alarms happen. For example, caregivers may sometimes fail to adjust alarm thresholds for individual patients due to the complex menu in the monitors. The absence of a standardized alarm menu structure may increase monitor complexity, and hence, some caregivers may tend to use default alarm settings. The default alarm settings that are based on population parameters are more likely to produce false alarms in critically ill patients whereas there may be an alarm setting that is better suited to the particular patient.

To assess a patient's status appropriately, cognitive integration of patient parameters and physiological parameters may be needed. Caregivers may fail to perform this cognitive integration due to several issues related to the current monitoring technology.

For example, the measurement of physiological parameters may be incorrect, for example, due to sensor-related or sensor-use problems. A correct interpretation of these artifacts may require an assessment of the overall context in which they have occurred.

In another example, nurses may be forced to process individual physiological parameters, both past and present, in a disconnected way to identify any inconsistencies between the patient's history and current status. This issue may necessitate a different display settings of the monitor specifically for the nurses.

ICU nurses may need information about recent interventions (e.g., drug administration) to be better integrated into trend displays on bedside monitors. When looking at trend data to assess the variability of parameters over time, information about recent interventions may not be directly available. Instead, such information may be recorded on the paper records or electronic charts, which may not be integrated into or accessible on the data monitor display. Such integrations might be obvious for an experienced caregiver but might not be apparent to a relatively inexperienced caregiver.

ICU nurses may experience issues of affordance, which gives them clues about which interaction with a monitor's interface is required to perform a particular operation. For example, they may experience difficulties in finding and selecting various functions on display menus and screens, which may result in many of the monitor's functions not being used. In a similar vein, there may be problems with the visibility of certain functions. For example, information about alarm settings may not always directly accessible.

Visibility may also be an issue in the context of the clarity of monitor function activation, where it may not always be obvious whether a particular function had been activated or deactivated by a previous user. Further, different users may prefer different settings of the monitor.

Some display monitors used in medical devices associated with a patient may have a lot of information 'clutter', making them difficult to use effectively.

Some display monitors include difficult-to-read text, which may increase the likelihood that certain numbers get misread. Abbreviated labels may also be misread, potentially leading to an incorrect assessment of patients' status and an increase in the time spent interacting with the monitor.

Some display monitors are complex such that there is a discrepancy between information provided on the monitor to guide nurses vs. knowledge that nurses needed to have previously acquired in order to navigate successfully through a menu (knowledge in the world vs. knowledge in the head). The menu structure may be "deep," making it difficult to find the desired information or menu item. Some patient monitor models may not provide help menus or a booklet of quick reference functions. Thus, some caregivers may experience difficulty with using some monitors, depending on their experience and knowledge.

As highlighted already, it may be difficult to perfectly adhere to formal data sharing and communication rules in complex and challenging environments. This may lead to patient information not being shared, shared in limited manner, misunderstood, or ignored.

In the ICU, a multidisciplinary team of caregivers may work together to manage patients by interpreting physiological parameters measured by the patient monitors. Each of these caregivers' way of patient treatment and preference of interacting with patient monitors, and hence use of the clinical data is different. These differences are dependent on multiple factors such as: the role, experience, workload, cognitive load of the caregiver, the hemodynamic stability of the patient, over crowdedness in the ICU, etc. In other words, the context, patient status, experience, and other personal factors make the analysis, interpretation, requirements of each caregiver different.

Due to the distributed availability of the patient data (via many devices, or stored at different places), caregivers may review these patient data individually or in small groups. The results of such analyses are typically communicated verbally (e.g., via phone) and rarely detailed notes are made. Even if some records are made, they just include the end results (e.g., suggestions), and do not include any information of the actions performed before arriving to the suggestions. In other words, the information of how a caregiver used and analyzed the patient data is not available.

Because of this unavailability of information related to the usage of patient data, it might so happen that to treat the same patient, different caregivers are looking at different datasets or different segments of the same data depending on their preferences. This gives rise to the following problems.

For example, unavailability of information may lead to clinical errors (e.g., contradictory, misunderstood, incomplete, wrong diagnosis and/or treatments), e.g. due to physician missing important information (for example, due to overconfidence), and the resident not questioning the physician due to thinking that the physician has seen the information because there are not any means to check how the physician used the patient data

Another example is the loss of time due to multiple people performing same type of analyses.

Another example is a false alarm, as a standardized (and not patient/user specific) alarm menu structure increases monitor complexity, and hence, the caregivers may tend to use default alarm settings

Another example is that sometimes a preferred settings/user interface of a patient monitor for one user might frustrate another user of the same or different role.

Additionally, not having a direct and easy access to how other clinical team members have utilized the patient data prevents caregivers learning from each other's actions. Having access to how an experienced caregiver used (e.g., for which analyses and simulations), and visualized (e.g. which parts of the data were paid the most attention) the patient data may help inexperienced caregivers to learn faster. For example, a first caregiver may notice that a second caregiver missed to analyze an important parameter, and can inform the corresponding person of its importance, which can help in preventing a potential clinical error.

### SUMMARY OF THE INVENTION

Certain aspects or embodiments described herein may relate to improving availability and/or access to information which could be relevant to a user involved in the care of a client such as a patient. Certain aspects or embodiments may address problems relating to a lack of understanding and/or knowledge about interactions between caregivers and clients.

In a first aspect, a method is described. The method is a computer-implemented method. The method comprises receiving a request associated with a user for information about a client. In response to the request, the method further comprises generating information indicative of at least one interaction between at least one caregiver and a client context associated with the client based on interaction data obtained responsive to the at least one interaction. The information is customized to the user based on a user profile associated with the user.

Some embodiments relating to the first aspect and other aspects are provided below.

In some embodiments, the interaction data is indicative of at least one of: a behavior of the caregiver as part of the at least one interaction; an activity of the caregiver as part of the at least one interaction; a response of the caregiver to the client context as part of the at least one interaction; a location of the caregiver as part of the at least one interaction; a time of the at least one interaction; equipment used by the caregiver as part of the at least one interaction; at least one type and/or setting of the equipment used by the caregiver as part of the at least one interaction.

In some embodiments, the generated information is indicative of a caregiver profile associated with the caregiver. The caregiver profile may comprise at least one of: a role of the caregiver; experience of the caregiver; historical data about the caregiver's interactions with: other clients; at least one other caregiver and/or the user; and/or a performance characteristic of the caregiver.

In some embodiments, generating the information comprises generating an interaction metric for quantifying the caregiver's understanding of a status of the client based on a predefined rule specifying how to generate the interaction metric based on at least one criterion associated with the at least one interaction.

In some embodiments, the method comprises using a statistical model to generate at least one interaction statistic based on the interaction data and/or at least one detected pattern in the interaction data. The method may further comprise generating the information based on the at least one interaction statistic.

In some embodiments, the method comprises using a status determining model to determine a potential status of the client based on the interaction statistic.

In some embodiments, the status determining model is configured to determine the potential status based on a client profile associated with the client and/or based on an analysis of the at least one interaction between the at least one caregiver and the client context.

In some embodiments, the method comprises identifying a point in a workflow considered to be relevant to a care plan. The workflow is associated with providing care to the client as part of the care plan. The method may further comprise providing the information to the user at the identified point.

In some embodiments, the method comprises, in response to receiving an alert associated with the client context, using a priority determining model to determine a priority level for the alert based on the interaction data.

In response to the interaction data being indicative of an expected cause of the alert that is not an event of concern, the method may comprise determining that the priority level is lower than if the alert was received in the absence of the interaction data.

In response to the interaction data being indicative of an event of concern, the method may comprise determining that the priority level is higher than if the alert was received in the absence of the interaction data

The method may further comprise indicating the determined priority level with the generated information.

In some embodiments, the method comprises determining whether the caregiver has specified whether at least part of the information is to be unavailable to the user. In response to determining that at least part of the information is to be unavailable to the user, the method may comprise preventing the specified part of the information from being made available via the generated information.

In some embodiments, the client context comprises a client device associated with the client. The client device comprises a user interface with which the caregiver interacts as part of the interaction. The method may comprise using the interaction data and/or the information to determine a preference of the caregiver for their interaction with the client device. In response to determining that the user interface does not meet the preference, the method may comprise causing the user interface to be modified to meet the preference.

In some embodiments, the method comprises accessing a care protocol. The method may further comprise identifying, based on the interaction data, a deviation from the care protocol and/or a measure of adherence to the care protocol. In response to identifying the deviation and/or the measure of adherence, the method may further comprise indicating the deviation and/or the measure of adherence via the information.

In some embodiments, the method comprises using a relationship identifying model to identify a correlation between at least part of the interaction data and a measurement of a health status of the client that is indicative of an existence of a relationship between the part of the interaction data and the measurement of the health status. The relationship identifying model may further be used to determine whether the part of the interaction data associated with the identified correlation is relevant to an outcome for the client based on a client profile associated with the client. In response to determining that the part of the interaction data is relevant to the outcome, the method may further comprise indicating such relevance via the information.

In a second aspect, a non-transitory machine-readable medium is described. The non-transitory machine-readable medium stores instructions which, when executed by processing circuitry, cause the processing circuitry to implement the method of any of the first aspect or related embodiments.

In a third aspect, apparatus is described. The apparatus comprises processing circuitry configured to communicate with an interface for receiving a request associated with a user for information about a client. The apparatus further comprises a machine-readable medium storing instructions which, when executed by the processing circuitry, cause the processing circuitry to, in response to the request, generate information indicative of at least one interaction between at least one caregiver and a client context associated with the client based on interaction data obtained responsive to the at least one interaction. The information is customized to the user based on a user profile associated with the user.

Certain aspects or embodiments described herein may provide various technical improvements in the context of, for example, managing, monitoring and/or understanding the caregiving associated with a client such as a patient.

These and other aspects of the invention will be apparent from and elucidated with reference to the embodiment(s) described hereinafter.

### BRIEF DESCRIPTION OF DRAWINGS

Exemplary embodiments of the invention will now be described, by way of example only, with reference to the following drawings, in which:
Figure 1 refers to a method of generating information indicative of an interaction according to an embodiment;
Figure 2 is a schematic drawing of a computing system for generating information according to an embodiment;
Figure 3 is a schematic drawing of a workflow for generating a metric according to an embodiment;
Figure 4 is a schematic drawing of a workflow for generating a statistic according to an embodiment;
Figure 5 is a graph representing statistical information for various interactions;
Figure 6 refers to a method of using an interaction statistic according to an embodiment;
Figure 7 refers to a method of providing information to a user according to an embodiment;
Figure 8 refers to a method of determining a priority level according to an embodiment;
Figure 9 refers to a method of determining whether information is to be unavailable according to an embodiment;
Figure 10 refers to a method of determining a preference according to an embodiment;
Figure 11 refers to a method of using interaction data according to an embodiment;
Figure 12 refers to a method of using a relationship identifying model according to an embodiment;
Figure 13 is a schematic drawing of a machine-readable medium for implementing various embodiments; and
Figure 14 is a schematic drawing of apparatus for implementing various embodiments.

### DETAILED DESCRIPTION OF THE EMBODIMENTS

As referred to herein, a 'client' may refer to an individual in receipt of care (i.e., a 'care receiver' such as a patient in a hospital or resident of a care home). This care may be in the context of diagnosis, treatment, health monitoring, rehabilitation, follow up and/or other care, whether in a care center such as a hospital, care home, hospice, etc., or in the community, such as at home.

As referred to herein, a 'client context' may refer to a client themselves, any equipment associated with the client such as medical devices, medical monitoring equipment, etc. and/or any data (i.e., 'client data') associated with the client such as medical records, vital signs, vital statistics, activity, nutrition, sleep, social interactions, and other activities of daily living, as well as data that may be generated by the clients themselves, such as written notes, recorded media (speech, video, audio), and other client generated data, etc.

As referred to herein, a 'caregiver' may refer to an individual involved in providing care to the client. At least one caregiver may be involved in providing care to a client. The skills and experience of a caregiver may differ, which may be apparent from interactions between the caregiver and the client context. Examples of caregivers include nurses, pharmacists, physicians, therapists, family members, friends, etc. Caregivers may interact with the client themselves or any equipment associated with the client e.g., as part of providing care to the client.

As referred to herein, a 'user' may refer to any person or team that has an interest in providing or managing the care given to a client. In some cases, the user may be a caregiver that interacts with a client, client data and/or equipment associated with the client (for example, medical monitoring equipment). In other cases, the user may analyze such interactions and/or make decisions on the care given to a client but may not necessarily be a 'direct' caregiver. A user may also refer to any person taking part in a formal or formal discussion about managing care provided to the client.

There may be multiple interactions between at least one caregiver and a client, especially in complex environments such as an ICU. Due to any of the aforementioned difficulties described previously, at least some information about these interactions may not be shared with other users (such as other team members involved in the care of a client). In some cases, such information may be relevant to the care of a client. For example, such information may indicate something that needs to be investigated. However, the other users may not be made aware of this information, potentially leading to an incorrect or less-than-optimum decision being made by the other users and/or lack of awareness of a problem that could be exacerbating the condition of the client. For example, this lack of effective information sharing may result in problems which could adversely affect the patient and/or fail to highlight compromised interactions with clients (by certain caregivers). Similar issues may arise in other caregiving environments such as in social care settings where a client may receive care.

Various embodiments described below may address at least one such issue.

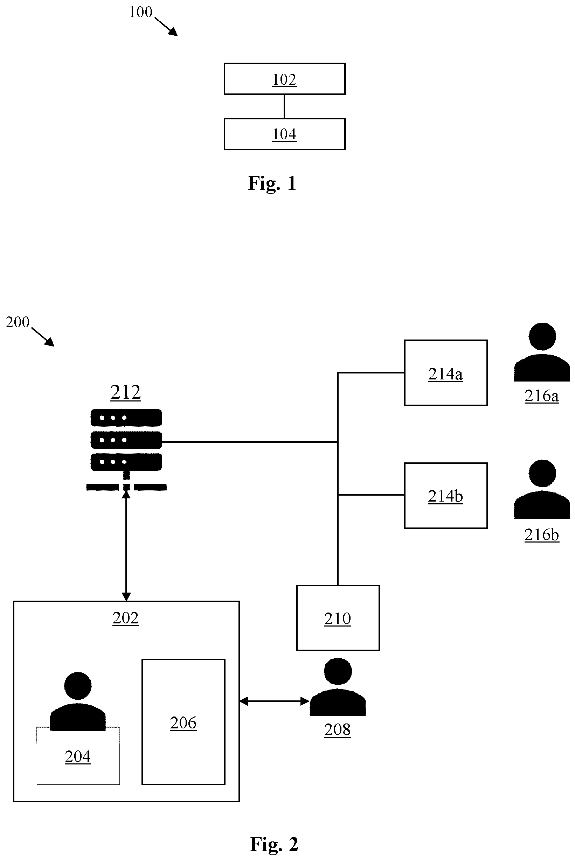

Figure 1 refers to a method 100 of generating information indicative of an interaction according to an embodiment. The method 100 is computer-implemented (e.g., by processing circuitry of a computing system that has access to stored data on interactions (i.e., 'interaction data') between at least one caregiver and a client). Details of the set-up of the computing system and other components for implementing various embodiments are described in more detail below.

The method 100 comprises, at block 102, receiving a request associated with a user for information about a client. The request may be generated by any relevant computing device associated with the computing system, and sent via a network to an entity that stored the information. The request may be associated with the user by virtue of having been generated as a result of user input to the computing device e.g., via a user interface of a computing device communicatively coupled to the computing system.

The request may indicate any relevant information about the user such as the user identity (such as their name, role, location, skills, experience, etc.) and/or particular information that the user is interested in (such as data about a specified client via a client identifier, a specified time period of interest that includes at least one interaction between at least one caregiver and the client and/or an indication about what information the user is interested in and/or how that information should be provided).

The method 100 further comprises, at block 104, in response to the request, generating information indicative of at least one interaction between at least one caregiver and a client context associated with the client based on interaction data obtained responsive to the at least one interaction. The information is customized to the user based on a user profile associated with the user.

As noted above, the interaction is with the client context, which may for example refer to the client themselves, client data and/or equipment associated with the client.

In the case of the client themselves, the interaction data could be based on a video feed, audio feed, etc., obtained by a data gathering device (e.g., a camera, smart phone, tablet, medical monitoring equipment such a patient monitor, or any other devices that may collect data about an interaction such as an ultrasound device (where the way the ultrasound device is used may generate "interaction data"), etc.) in the vicinity of the client and operable to sense the interaction visually, audibly, etc. For example, the data gathering device could record the interaction and send the recorded data to the computing system mentioned above. In another example, the recorded data may be processed by the data gathering device and the processed results (e.g., features, metrics, statistics, etc.) can be sent to the computing system.

In the case of the client data, the interaction data could represent how the caregiver interacted with the data itself e.g., which data was accessed, for how long the data was accessed, what analysis was performed, etc.

In the case of the equipment associated with the client, the interaction data could be generated (e.g., by processing circuitry of the equipment) responsive to the caregiver's interaction with the equipment (e.g., a user interface of such equipment such as with buttons, a screen, etc. and/or how that equipment was used with respect to the client). Such an interaction may refer to, for example, a record of which menu(s) were accessed by the caregiver, which settings were used, how long the caregiver interacted with a certain function of the medical equipment, which function(s) were used, how the equipment was used on client, where on the client a part of the equipment was positioned, how fast it was moved, for how long was it used, etc.

The generated information may take various forms such as raw data associated with the at least one interaction (e.g., as generating by a data gathering device), a metric derived from raw data, a statistic derived from the raw data, an interpretation of the at least one interaction and/or any other data of interest that may quantitatively and/or qualitatively represent the at least one interaction between the at least one caregiver and the client.

As referred to above, the generated information is based on interaction data obtained responsive to the at least one interaction. Examples of such interaction data include any data collected that may be indicative of the interaction between the caregiver and the client context. This data can be collected in any way such as automatically by a data gathering device such as a sensor, processing circuitry, an embedded system, etc., configured to detect/monitor and interaction and/or produce interaction data responsive to the at least one interaction. Such interaction data may be stored in any appropriate format in a memory device accessible to the computing system (such as a database). The interaction data may be associated with the client and/or the client context (e.g., via a client identifier) such that the request (which may include the client identifier) can be linked to the correct interaction data (i.e., the interaction data associated with the identified client). The format of the interaction data may therefore depend on the source of the interaction data.

The format of the generated information may be in accordance with the needs of the user and/or the implementation of a computing device used to receive and/or interpret such generated information. For example, the generated information may comprise character strings, codes, numbers, image data, graphical data, etc., representing events or activities that have occurred as part of the at least one interaction. As part of generating the information, the stored interaction data may be transformed in such a way that it is customized to the user.

As referred to above, the information is customized to the user based on a user profile associated with the user. A user profile may refer to any characteristic of the user such as their status, job role, skills, experience, the context of the user (for example, in certain situations, such as emergency, when physicians have delegated some of their task for experienced nurses, the nurse may be able to access physician views), etc. Thus, as part of generating the information, the information may be generated in such a way that it is customized to the user based on their user profile. This may increase the relevance of the information generated for the user. For example, certain users may be interested in certain information but not certain other information. Based on the user profile (e.g., as indicated by the request or accessible to the computing system via a user identifier), the computing system may generate the information that is relevant to the user. In other similar words, certain users may find certain information useful whereas other users may not find that information useful. By customizing the generated information to the user, the user may have improved access to more relevant, reliable and/or accurate information about the at least one interaction. Further, the user may have faster access to the relevant data, which may help with faster decision making.

The customization may be based on a rule (e.g., defined by instructions readable and executable by the processing circuitry) that specifies which interaction data might be of relevance to the user, based on their user profile (and, in some cases, the client status such as their condition where a different client status may lead to a different customization), as well as specifying how such interaction data should be provided to the user in the form of the 'generated information'. In other words, the customization need not be static, and can be also dependent on the user context and/or client status. In an example, the rule may associate a job role with certain information that might be useful for users with the job role. Certain interactions may be of higher interest to the user since they may have a high relevance to any clinical decisions that the user has to make. In this case, the generated information may comprise raw data, metrics, statistic(s), etc., derived from the interaction data associated with the higher-interest interactions. In another example, the rule may specify the format of the generated information based on the user profile, for example, so that the user can understand the generated information. In another example, the rule may take into account the previous interactions of the (same) user.

Certain embodiments described herein, including method 100 and various other embodiments, may provide various technical improvements in the context of, for example, managing, monitoring and/or understanding the caregiving associated with a client such as a patient.

In some cases, certain embodiments described herein may improve the reliability, accessibility, relevance and/or accuracy of data about a client that a user can access. Such information sharing may lead to at least one of: an improved outcome for the client, an improvement in the way that caregivers interact with clients, an improvement in terms of ease of access to potentially relevant information in various scenarios and/or making data or analysis available that would not otherwise be available or accessible to the user.

In some cases, certain embodiments described herein may make it easier to find and/or visualize caregiver related information, reduce withholding or forgetting of information, minimize information gaps (e.g., among team members), make it easier to find and visualize the focus on the current client (patient) care, make it more likely that other client care aspects that have not received attention are highlighted and/or address any problem described herein. This may make client care more effective (e.g., shorter stay in ICU and lower mortality), lead to faster decision making and/or reduce clinical errors (e.g., resulting from poor team interactions).

In some cases, certain embodiments described herein may be facilitated by (i) quantifying the interaction of caregivers with a client (e.g., patient) context, (ii) storing and/or dynamically updating the information about the interactions and/or (iii) making the stored interactions available to all the caregivers, for example to whoever is examining the client. Making the information easily available (and up-to-date) may assist the user with a task relating to caregiving such as making a clinical decision.

In some cases, certain embodiments described herein may implement at least one of the following functionality: (i) recording caregiver-related client data usage as metadata and associate such data with the client data (e.g., the stored interaction data and/or information may have a link to an identified client), (ii) use the recorded metadata to quantify the actions of the caregiver with respect to their usage of the client data, (iii) provide the metadata and/or information extracted from the metadata based on the needs of a team (e.g. per team members), and/or (iv) provide the metadata and/or information extracted from the metadata based on client status (e.g., per client data type). In this manner, client data may 'follow' the client, which may help to ensure continued access to data even when different caregivers and users are involved with the client.

A system for implementing various embodiments described herein, including method 100 and other embodiments, is now described.

Figure 2 is a schematic drawing of a system 200 for generating information according to an embodiment.

The system 200 is associated with a client context 202 which, in this embodiment, comprises a client 204 and equipment 206 associated with the client 204. A caregiver 208 may interact with the client context 202 as part of providing care to the client 204. As part of or as a result of this interaction, 'interaction data' may be obtained, as explained below.

In some cases, the equipment 206 may comprise medical equipment for use in monitoring and/or caring for the client 204. In some cases, this medical equipment may be used for gathering 'interaction data' about the interaction between the client 204 and the equipment 206.

In some cases, the equipment 206 may comprise a data-gathering device for use in gathering the 'interaction data'. For example, the equipment 206 could be a camera and/or microphone for gathering visual and/or audio data indicative of the interaction.

In some cases, the caregiver 208 may possess their own data-gathering device 210 for use in gathering the 'interaction data'. This data-gathering device 210 could be any computing device capable of collecting data such as visual and/or audio data about the interaction such as a smart phone, tablet, etc. In some cases, the data-gathering device 210 may permit the caregiver 208 to actively enter data about the interaction. In some cases, the data-gathering device 210 may passively obtain data about the interaction.

Any number and combination of these different types of devices for gathering the interaction data may be used. Although one caregiver 208 is depicted, there could be multiple caregivers involved in providing care to the same client 204 (e.g., at the same or different times).

The system 200 further comprises a computing system 212 for at least one of: receiving interaction data (e.g., from the equipment 206 and/or the data-gathering device 210), storing of the interaction data, handling the request and generating information. The computing system 212 may be implemented by a remote or local server, in the cloud, by a local computer in the vicinity of the client context 202, etc. The processing circuitry referred to in the method 100 and other embodiments described herein may be implemented by the computing system 212.

As depicted by Figure 2, the client context 202 (e.g., via the equipment 206) and the data-gathering device 210 are communicatively coupled to the computing system 212. However, depending on the set-up, one or both of the client context 202 and the data-gathering device 210 may be communicatively coupled to the computing system 212.

The computing system 212 may obtain interaction data associated with other clients (not shown). This interaction data may be tagged and/or stored in such a way that the interaction data is linked to the identity of the client 204 (such as via a client identifier or in a 'client file', which could link to the client's 204 medical records). Thus, when the information is generated, the information may be based on the correct client 204, as indicated by a client identifier indicated by the request.

The system 200 further comprises a user computing device 214a associated with a first user 216a and another user computing device 214b associated with a second user 216b. The user computing device 214 may be any computing device that can be communicatively coupled to the computing system 212 such as a smart phone, tablet, personal computer, laptop, etc. The user computing devices 214 may or may not be communicatively coupled to each other and/or the data-gathering device 210, depending on the set-up. The number of user computing devices 214 and users 216 also depends on the set-up. Two user computing devices 214 and two users 216 are shown to assist with explanation of how the generated information is personalized to each user 216. As noted previously, a user 216 may be synonymous with being a caregiver 208, however, roles may vary within a team. For example, certain caregivers 208 may interact more often and/or more directly with the client context 202, which may lead to certain caregivers 208 not having a full understanding of the interactions. If any caregiver 208 accesses the interaction data/generated information, they may be considered a 'user' 216.

In an example implementation of the system 200, the user 216a requests information about at least one interaction between the client context 202 and the caregiver 208 by causing the user computing device 214a to send such a request to the computing system 212. The request may comprise or point to a user profile associated with the user 216a. The user profile may indicate that the user 216a has role 'A'.

Similarly, the user 216b requests information about at least one interaction between the client context 202 and the caregiver 208 by causing the user computing device 214b to send such a request to the computing system 212. The request may comprise or point to a user profile associated with the user 216b. The user profile may indicate that the user 216b has role 'B'.

The computing system 212 generates information that is customized to each user 216a, 216b, for example, based on the 'rule' mentioned above. The generated information may be relevant to the user role, 'A' or 'B' such that the generated information (which may be sent by the computing system 212 to the respective user computing device 214a, 214b) is different for each user 216a, 216b. For example, the generated information may comprise raw data, a metric, statistic, etc., which is relevant to each user 216a, 216b depending on their role.

Interaction data which may not otherwise be collected (e.g., in the formal rounds) may be obtained and stored in the computing system 212 (or in a memory device accessible to the computing system 212) for future use in generating the information. The set-up may ensure that the interaction data, and hence the generated information, is correctly linked to the client context 202. Thus, the users 216 may obtain information about any client context 202 when needed. Further, the users 216 may have improved access to more relevant, reliable and/or accurate information about the at least one interaction.

The following description refers to various implementations of embodiments with reference to the system 200 of Figure 2.

In some cases, caregiver-related metadata may be associated with the client data (which may be stored in the computing system 212) e.g., by attaching or otherwise linking the metadata to the client data. The metadata may refer to an action performed with respect to the client data. For example, for a particular client data (e.g. a medication given, and the vital signs monitored afterwards), the nature of the interaction with that client data may be associated with the client data (e.g., via the client identifier mentioned previously) as metadata.

In some cases, at least one of: the identity, role and/or experience level of the caregiver 208 (i.e., part of a 'caregiver profile') who visualized the client data may form at least part of the metadata.

In some cases, the interaction and/or duration of visualization of the client data may form at least part of the metadata. By way of example, this may refer to what the caregiver 208 is looking at with respect to the client data, how often the caregiver 208 is looking at the client 204 and/or how much attention is paid by the caregiver 208 to contextual parameters such as forming part of the client profile but not necessarily directly related to a particular health issue (e.g., a risk factor such as age, weight, etc.). For example, a caregiver 208 that pays less attention to certain contextual parameters may be useful data because the user 216 may be better informed about the interaction.

In some cases, certain actions performed, such as zooming and annotations in certain regions of a user interface (e.g., of the equipment 206) may form part of the metadata. Such metadata may indicate the effectiveness of the caregiver 208 in terms of their interaction with the equipment 206, which may be useful information for the user 216.

In some cases, certain uses, such as using the client data as input to a software (e.g., accessible via the equipment 206 and/or data-gathering device 210) such as simulation tool, analysis software, caregiver decision support software or digital twin may form part of the metadata. This may indicate to the user 216 whether the software is being used in an effective way and/or point to potentially inaccurate results due to the interaction with the software.

In some cases, the stored metadata may be used to calculate a metric indicating the level and/or type of interaction of the caregiver 208 as indicated by the metadata. Examples of metrics include the percentage of data viewed (e.g., the metric may indicate how much client data/metadata of a total available amount of data the caregiver 208 viewed as part of their interaction with the client context 202), the duration of viewing such data, the type of analyses performed with the data, whether or not a predefined checklist has been completed.

In some cases, the generated information may comprise or at least be representative of the metadata based on the needs of the user 216 (and such information may be provided in any appropriate format such as visually or audibly). For example, during the formal rounds, the generated information may comprise statistical data per caregiver 208, e.g., indicating how they used the client data during their own analyses. Further, the generated information may help to better understand to where the caregiver 208 dedicated their attention. These may lead to a better 'team' understanding of how the various caregivers 208 have interacted with the client context 202 and whether improvements can be made and/or whether this information is indicative of an underlying, potentially undiagnosed, problem with the client 204. In some cases, a "team" version of the information may be generated, for example, by aggregating, filtering and/or combining all interaction information for team members. In other similar words, the team version of the information may be used to represent/visualize how different users interacted with the client context. In some cases, the generated information may comprise an indication/calculation representative of similar measures per groups of people (e.g. clinical teams).

In some cases, the generated information may be based on a need according to the status of the client 204. For example, the generated information may refer to statistic of how different types of client data were used by different caregivers 208. In another example, the generated information may indicate that the client data is critical (depending on client status), and calculate metrics showing if the client data has received sufficient attention (e.g. examination time) from the different caregivers 208 in a team. For example, if medication with strong side effects is administered to the client 204 but this not monitored by a pharmacist, in addition to the intensivist, the lack of involvement of the pharmacist may be a flag or concern.

Certain parts of client data associated with the client context 202 may be relevant to various users 216 involved in providing care to the client 204. If a certain team member/caregiver 208 does not 'interact' at all or interacts in a certain way with the client context 202 then this may be a flag or a concern. Associating this 'metadata' with the client data and/or client context 202 may lead to a better contextual understanding of the client data and/or client context 202. In other similar words, any interaction with the client context 202 (including with the client 204 and/or their client data) may be recorded as 'metadata' associated with the client 204 and this 'interaction data' be stored for future use e.g., for generating the information.

Some implementations relating to the above are now described.

In some cases, the interaction data and/or the generated information may be used to modify the functionality of and/or improve access to functionality of the equipment 206. For example, a certain function may be accessed frequently by the caregiver 208 (e.g., by the particular caregiver 208, caregiver 208 in this role and/or for such a client 204). The generated information may be used to control the equipment 206 e.g., by automatically adjusting its settings so that a certain function is easier to access. Possible implementations include making a shortcut on a user interface (e.g., screen) of the equipment 206 to the particular function and/or providing access to the generated information (e.g., for the caregiver 208 based on the caregiver profile).

In some cases, the use of the interaction data and/or the generated information may be used (by the equipment 206) to make a "popular" or "important" feature or functionality more prominent or more easily accessible (e.g., with fewer "clicks" or "taps") within a user interface of the equipment 206. In some cases, the accessibility level of an existing functionality may be changed based on an understanding of the caregiver interaction with the equipment 206. There may be various ways to change the accessibility level, for example, by adaptation of the interface (e.g., a graphical user interface), by ranking certain functionality higher (for example, for search results), by realizing the functionality earlier than others (e.g. running the identified function first, or presenting its results earlier), allocating more resources (e.g., memory, storage, permissions) to a given function so that it can be run faster, more often, or by a different number of individuals at a given time.

In some cases, the interaction data and/or the generated information may enable the development of an education tool (e.g., for use in a different setting such as when a caregiver is undertaking training or not during the caregiver's shift). In retrospect, caregivers 208 can take guided tours through different client pathways and learn from activities from other caregivers 208. It can also be used to perform retrospective analyses to evaluate if optimal care was provided to a client 204, and capture learnings and recommendations to adjust certain processes/standard operating procedures (SOPs). The improved access, relevance and/or accuracy of the interaction data and/or generated information may provide insights that might not otherwise be derivable from the data made available by other means such as in the formal rounds.

In some cases, the system 200 may be used to attribute an importance indicator to client-caregiver interactions. By correlating 'interaction data' with vital signs data, actions critical for client outcome can be identified and highlighted, whereas less important actions can be listed as candidates for further investigation to make certain processes leaner.

Certain embodiments described herein may quantify a caregiver's 208 interaction with client data residing at several sources/systems (e.g., equipment 206 such as a patient monitor, client data such as in electronic medical records (EMRs) and/or the data-gathering device 210) connected in the system 200. The transition of client monitoring data towards mobile and personal monitoring devices may enable unobtrusive capturing of many new types of data (such as how caregivers 208 are using and interacting with the client context 202) that are presently difficult to acquire in the bedside monitors (e.g., due to infrastructural issues).

The interaction data may be captured in an unobtrusive or passive manner and may be used to quantify the interaction of the caregiver 208 with the client context 202. The following refers to possible interaction data captured in this manner. In some cases, duration of a certain signal/data, or signal element (i.e., both forming part of the 'interaction data') may be visualized and studied (e.g., how long the caregiver 208 interacted with any particular part of the client context 202). In some cases, the number and/or region of zooming and un-zooming actions that occurred as part of the caregiver's 208 interaction with the client context 202 (e.g., with respect to a user interface of the equipment 206). In some cases, the signal/data elements may be visualized together, software-based analyses performed (e.g. which simulations) and/or follow-up actions carried out based on the interaction data. In some cases, the interaction data may comprise 'input' (e.g., a type, amount and/or content of the interaction data, including both text and spoken - determined automatically by natural language processing (NLP) methods) entered by the caregiver 208 (such as comments, data annotations, notes, etc.). In some cases, the interaction data may comprise or be based on verbal and/or non-verbal interaction between the caregiver 208 and the client 204. In some cases, the interaction data may comprise or be based on verbal and/or non-verbal interaction between the caregiver 208 and the user 216.

As mentioned already, the generated information may comprise statistic(s) representative of the interaction data (e.g., derived from at least one caregiver 208 interaction). The statistic may quantify, simplify or otherwise emphasize patterns in the interaction(s). Any reference to a statistic described herein may refer to 'at least one' (interaction) statistic.

In some cases, interaction statistic (e.g., in terms of how the client data is stored, accessed, searched, stored in the EMR) derived from interactions between client contexts 202 and experienced users 216 can be used by other caregivers 208 to treat/care for clients at a higher quality. In some cases, interaction statistic may provide an easy way to report a condition of a client 204 by synthesizing the observed data and observation patterns to form hypotheses instead of simply reporting the streams of raw data to caregivers 208. In some cases, interaction statistic can be used to improve models on 'patient similarity' such as indicating how certain classes of patients may response to treatment/care/interactions. In that way, a 'patient similarity' model may be determined not only based on the 'multidimensional' client data but also depending on the treatment/managing actions as indicated according to the interaction statistic. Thus, the multidimensional patient data can then be transformed into actionable information together with the situational awareness to caregivers 208. In some cases, specific client information could be optimally presented to promote not only a better understanding of the client's pathophysiologic state and support decision-making but also to facilitate collaboration (consultation, teleconsultation) and workflow (handover, treatment) management among the team/s.

In some cases, the interaction data can be used to perform focused/selective signal feature extraction, and analyses of client data. As an example, if it appears that experienced caregivers 208 mostly investigate the trend of a certain parameter, as opposed to the absolute value of the parameter, the trend information could be emphasized more in other similar client 204 cases. In other cases, the caregiver 208 may be interested in the magnitude of the variation of a signal. This could trigger a different visualization in which features regarding signal variation may be optimally displayed. In other similar words, patterns apparent in the interaction data may be represented by the 'generated information', which may be used to inform/control how equipment 206 functions to assist caregivers 208 (e.g., interaction data associated with experienced caregivers 208 or users 216 may be used to help, inform or otherwise direct less experienced caregivers 208 to interact with the client context 202 in a particular manner).

Some embodiments relating to the above are now described.

In some embodiments, the interaction data is indicative of at least one of: a behavior of the caregiver 208 as part of the at least one interaction; an activity of the caregiver 208 as part of the at least one interaction; a response of the caregiver 208 to the client context 202 as part of the at least one interaction; a location of the caregiver 208 as part of the at least one interaction; a time of the at least one interaction; equipment 206 used by the caregiver 208 as part of the at least one interaction; at least one type and/or setting of the equipment 206 used by the caregiver 208 as part of the at least one interaction.

Various implementations of such embodiments are now described.

In some cases, the behavior of the caregiver 208 may refer to the manner in which the caregiver 208 interacts with the client context 202. By way of example, the personal interaction with the client 204 may indicate something interesting e.g., if the caregiver 208 notices something that could be relevant to the client's 204 care but they are not trained or aware of its significance. In an example, the equipment 206/data-gathering device 210 comprises a camera for monitoring the interaction. The camera may collect a video feed from which it is possible to track the eye, facial and/or hand movement of the caregiver 208 and/or the client 204 as part of the interaction. Such 'tracking' interaction data may be indicative of something of clinical relevance that can be highlighted to the caregiver 208 and/or the user 216. Similarly, a lack of eye/face/hand movement where such movement might otherwise be expected could raise a flag so that the user 216 can investigate further.

An example scenario where the lack of personal interaction with the client 204 may indicate something interesting may be if the system 212 noticed that the client 204 has a drainage and/or the client 204 may be having a hemodynamic shock condition. The hospital standard operating procedure (SOP) for drainage management may indicate that the wound dressing and the draining needs to be checked at the minimum 3 times a day and provides the correct sequence of steps that need to be taken. In this example, the camera 210 may indicate that there is no interaction or an inappropriate interaction of the caregiver 208 with the client context 202 and/or there is interaction but this is deviating from the SOP sequence of steps. The need to do drainage management can be highlighted to the caregiver 208 and/or the user 216.

In some cases, the activity of the caregiver 208 may refer to the type of medical procedures carried out, whether or not the equipment 208 is used in a certain way, time spent performing the activity and/or any caregiving activity, whether in the vicinity of the client 204 or not (e.g., interacting with the client data may still refer to interacting with the client context 202).

In some cases, the 'response' of the caregiver 208 may refer to a result of the interaction which could be indicative of something of interest. In an example, the response may refer to how the caregiver 208 reacts to speech, sounds or other signals. In another example, a 'surprised' or 'confused' look on the caregiver's 208 face (as examples of active or subliminal reactions to the client context 202) may indicate something amiss with the client 204. However, the occurrence of such responses might normally be missed from the formal rounds. However, the interaction data recorded may highlight such responses which could be of clinical significance and potentially lead to an improved outcome for the client 204.

In some cases, the location and/or time of the interaction may be included in the interaction data. The location may refer to, for example, whether the caregiver 208 was in the vicinity of the client 204 as part of the interaction.

Not all interactions with the client context 202 may be in the vicinity of the client 204 themselves (e.g., interactions with the client data and/or equipment 206 may not involve a direct interaction with the client 204). In another example, the interaction may have happened by the client 204 bedside via a patient monitor, or in physician's room via a personal computer for accessing the monitor data and EMR. Knowing the location of the interaction might influence the weight given to the different interaction data. For example, if is established that the caregiver 208 is on the bedside and did not visualize all client data, this may be acceptable because most probably they received other information via other means such as verbally, or by own examination. On the other hand, if the location is office room, it may not be acceptable that the caregiver 208 did not visualize a particular client context 202 information/data.

With respect to 'time', the time may be indicative of various contextual information. For example, certain events may occur at certain times and knowing the times could be indicative of something of clinical interest. For example, knowing that a caregiver 208 examines client data after medication administration can be useful, because it can indicate that a particular type of response was expected. In another example, knowing the time of day might be indicative of a fatigue level of the caregiver 208 e.g., based on the shift time, which could be of relevance to a clinical response.

In some cases, the particular configuration of the equipment 206, software associated with the equipment 206 and/or user interface characteristics may be taken into account as an example of interaction data. For examples, some devices/software/user interface or environments (e.g. internet connection) may not allow the caregiver 208 to access all features or functionality that could be relevant to the client context 202. In other words, knowing what data, features and/or functionality is available for access, and then evaluating the interaction based on the availability of the data, features and/or functionality may be useful for better understanding the interaction.

In some embodiments, the generated information is indicative of a caregiver profile associated with the caregiver 208. In this case, the caregiver profile comprises at least one of: a role (e.g., job) of the caregiver 208; experience of the caregiver 208; historical data about the caregiver's 208 interactions with: other clients 204; at least one other caregiver 208 and/or the user 216; and/or a performance characteristic of the caregiver 208. The generated information may comprise the caregiver profile, point to the caregiver profile (e.g., stored elsewhere) or otherwise indicate information about the caregiver and represent such information in a suitable way for the user 216.

In some cases, the 'performance characteristic' could refer to the ability of the caregiver 208 e.g., how well the caregiver 208 performs at their job. A potential issue with any criteria relating to the performance characteristic could be flagged when generating the information. This information could include a score for the caregiver 208. For example, a particularly experienced or high performing caregiver might have a higher 'performance score' and their interaction with the client context 202 might be considered to be highly valid (with less need to 'double check') than a lower performing caregiver 208.

The interaction may refer to the interaction between the caregiver 208 and the client context 202. However, in some cases, the interaction data may be representative of the interaction between the caregiver 208 and other caregivers 208. Again, this may be indicative of team dynamics, which may help with understanding and facilitating matching between various caregivers 208.

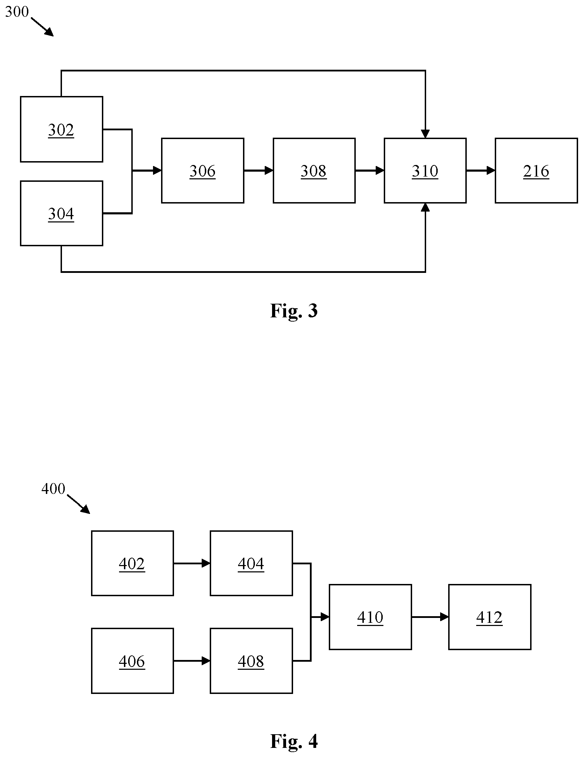

Figure 3 is a schematic drawing of a workflow 300 for generating a metric according to an embodiment. Reference is made to Figure 2 in the description of the workflow 300. The workflow 300 may be at least partially implemented by the computing system 212, as referred to below. The workflow 300 may generally refer to the obtaining of the interaction data, generating metadata (e.g., with respect to a predefined template stored in or accessible to the computing system 212 in which certain interaction data is stored in a particular format, level/type of metadata, etc.) and generating information in the form of a (e.g., at least one) metric.

According to the workflow 300, at block 302, equipment 206 such as a client monitor may generate/collect 'interaction data'.

At block 304 of the workflow 300, the client 204 EMR (i.e., 'client data') may be accessed and used to provide information on the client profile.

Data from one or both of blocks 302 and 304 may be extracted, at block 306 of the workflow 300, when monitoring interactions between the caregiver 308 and the client context 202.

The extracted 'interaction data' may be stored, at block 308 of the workflow 300, as 'metadata' in or accessible to the computing system 212 (such as raw data, in a predefined template, etc.).

In response to a request, a metric based on this metadata may be generated (i.e., 'generated information') at block 310 of the workflow 300. Such a metric may be dynamically generated e.g., it may change in response to any changes to the metadata. As depicted by the workflow 300, the resulting metric may be forwarded to the user 216.

As also depicted by the workflow 300, the interaction data and/or the client data may be used to directly generate the metric at block 310. One or both of the 'raw' data and the metric may be forwarded to the user 216, if such data/information is useful for the user 216.

Some further details of various implementations of the workflow 300 are described below.

In some cases, the metadata to be stored may be predefined initially as part of the set-up of the computing system 212. The definition of the metadata to be stored can be done as a hospital policy (e.g., similar to the checklists that are prepared by the hospital itself). For example, an ICU team can decide what type of metadata they want to be recorded. Various metadata that can be recorded include the following.

In some cases, the duration of an interaction by a caregiver 208 may be represented in the metadata (for example, this could be determined automatically based on the active caregiver 208 interface/screen on the equipment 206 used for visualization and/or caregiver 208 clicks with a mouse or touches on touchscreen display). In some cases, metrics such as frequency of visiting a certain page on the screen (e.g., how often is a caregiver 208 returning to a certain page, which may also be used to optimize the hierarchy/structure of all pages), duration-between-interactions, timestamp of interaction (e.g. due to pattern difference during day/night as some caregivers report during a shift).

In some cases, notes, or annotations performed on the data (e.g., indicated as yes/no) may be represented in the metadata (for example, such metadata may be determined based on annotations done directly on the data and/or or based on natural language processing where caregiver 208-entered text is analyzed).

In some cases, data acknowledged (e.g., by use of a checklist) by the caregiver 208 (for example, determined based on the caregiver 208 filling related checklists) may be represented in the metadata.

The above are just a few examples, and a caregiving team, user, etc., may decide to record other metadata information. Various combinations of such metadata may be stored for use in generating information, including metrics, statistic, etc.

The metadata stored for different data types (e.g., medication, vital signs, lab data), or from different data presentation modalities (e.g. patient monitors, caregiver computing devices, EMR) can be different. Moreover, the metadata template can be also different for different patient groups, or patient criticality.

Once the metadata template is defined, the interaction of the caregivers 208 with the client data, and the devices/equipment 206 and mediums that provide access to client data may be monitored and/or analyzed with the purpose of determining the metadata. Since the needed information may be well defined, this process can be implemented in a straightforward manner if it is already known how the client data is typically accessed and studied by the caregivers 208. For example, for a physician that makes use of their personal computing device (such as a smart phone, tablet, etc.) to access the client data, personal device usage can be a source of metadata (e.g., similar to how websites collect user browsing, and clicking data, the physician's interaction with data can be recorded). For a nurse, which would typically use patient monitors, the reactions to the alarms generated by the patient monitor, and different visualization screen of the monitor can be used to gather the metadata from the nurse.

As referred to above, the collected metadata may be analyzed (at block 310) to calculate an interaction metric per client data type and per caregiver 208, and/or per client 204 and per caregiver 208. In some cases, this can be a binary variable indicating if the caregiver 208 is sufficiently informed (e.g., of the current client condition/status) or not. In some cases, a fuzzy (or probabilistic) variable can be calculated, which can have any value between 0 and 1, depending on the estimated familiarity of the caregiver 208 with the patient data and/or condition. Thus, analysis of such metadata may yield useful information about the interaction, which can be provided to the user 216 so that the user 216 can become more aware about the interaction.

The interaction metric calculation may be dynamic because it may depend on the available client data, and client condition/status. When new metadata becomes available (e.g., if the client condition changes), the metric may need to be updated.

In order to calculate the metric, a rule based approach may be followed in which a checklist is evaluated to determine how many of predetermined criteria are followed (e.g., a percentage of the checklist completed may be an example of a metric) and/or a comparison against a predefined threshold may be evaluated (e.g., how much time a caregiver 208 spent on an activity may be evaluated with respect to the threshold - if below the threshold, this may indicate that not enough time was spent and the metric may be binary or some other indicator of the interaction). In some cases, different rules for different specializations can be used. For instance, for a clinical pharmacist the rule may be used for acknowledging the medications taken, and analyzing of the post-medication vital signs recorded for at least a specified period of time such as 60 seconds. For an intensivist, the rule can be visualization of all client data recorded in a specified period of time such as the last hour for at least a specified period of time such as 60 seconds, and using the interaction data and/or client data to run a simulation to estimate the risk of various medical scenarios.

In some cases, calculating the metric may involve quantifying the data access and use pattern of the client data by the caregiver 208. This can be achieved, for example, by establishing when the caregiver 208 is using personal computing device or account and/or tracking can be used, as described previously.

Thus, in some embodiments, generating the information comprises generating an interaction metric for quantifying the caregiver's 208 understanding of a status of the client 204 based on a predefined rule specifying how to generate the interaction metric based on at least one criterion associated with the at least one interaction. The predefined rule may refer to the 'rule' described previously. The criterion may refer to what data from the at least one interaction to use in order to generate the interaction metric. For example, the at least one criterion may comprise a measure (i.e., a qualitative and/or quantitative measure) derived from at least one of: a time/duration of the interaction, location of interaction, nature/type of the interaction activity, which activity or activities were carried out as part of the interaction, a response of the caregiver 208, which equipment 206 was used by the caregiver 208, which type and/or setting of the equipment 206 was used by the caregiver 208, etc. Thus, in these and other examples, the criterion may refer to some measure of these interactions and the interaction metric may be based on such criteria (e.g., a numerical indicator of such interactions).

Figure 4 is a schematic drawing of a workflow 400 for generating a statistic according to an embodiment. Reference is made to Figure 2 in the description of the workflow 400. The workflow 400 may be at least partially implemented by the computing system 212. The workflow 400 may be related to the workflow 300, which refers to generating a metric, which could be used as part of generating the statistic(s) in workflow 400.

At block 402 of the workflow 400, a first caregiver 208 is monitored and 'interaction data' obtained (and stored as metadata, as referred to in Figure 3).

At block 404 of the workflow 400, the first caregiver's 208 interaction data is used for generating the information, where the information comprises the statistic about the interaction data (associated with the first caregiver 208).

At block 406 of the workflow 400, a second caregiver 208 is monitored and 'interaction data' obtained (and stored as metadata, as referred to in Figure 3).

At block 408 of the workflow 400, the second caregiver's 208 interaction data is used for generating the information, where the information comprises the statistic about the interaction data (associated with the second caregiver 208).

At block 410 of the workflow 400, the interaction statistic associated with each caregiver 208 is collated and used to generate 'overall' statistic (which may also refer to 'generating information' as described herein).

At block 412 of the workflow 400, the overall statistic is used for various applications such as at least one of: EMR data sorting, labelling, alert/alarm generation, adapting an interface of the equipment 206 and/or adapting a computing device associated with the caregiver(s) 208, etc.

A statistical model, accessible to the computing system 212, may be used for generating the statistic. Such a statistical model may be configured to detect patterns in the interaction data and/or evaluate the interaction data and/or extract data values which can be used to construct the statistic. For example, quantified interaction data such as amount of interactions/time of interactions associated with each caregiver 208 may be represented in a statistical form such as a comparison of time spent per caregiver 208 e.g., based on their role. The pattern(s)/statistic may be deduced from raw data, metadata, client data, etc. based on the statistical model, which may be configured to extract certain data values and generate the statistic based on such extracted data values.

The interaction statistic, as described in more detail below, can be dependent on multiple factors e.g., client condition, caregiver's 208 experience, crowdedness, fatigue levels, handover scenario, etc. Each of these factors may have associated data values which can be evaluated and used for constructing at least one statistic.

Some example statistics are described below.

In some cases, the statistic may represent frequency of observation. For example, it may be possible to evaluate the frequency of observation made by the caregivers 208 at each of the parameters (as described below).

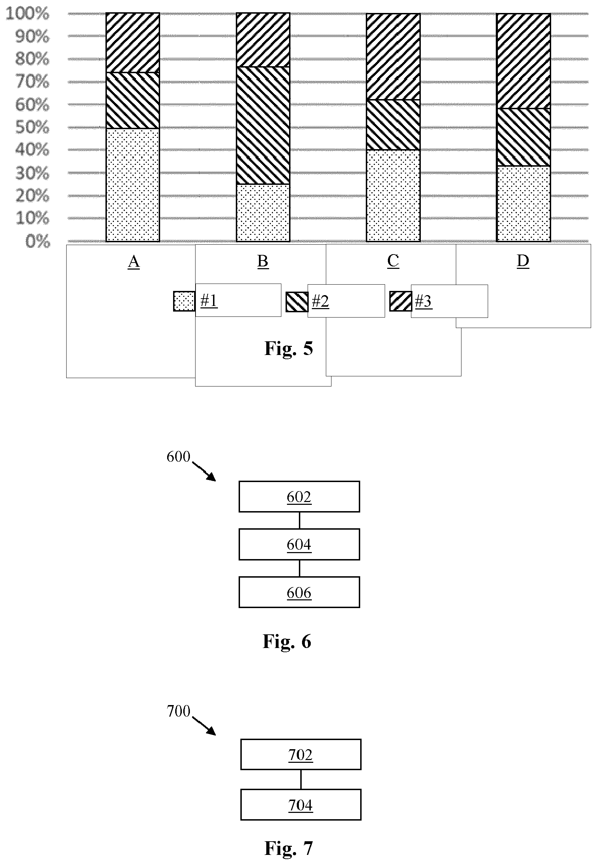

Figure 5 is a graph representing statistical information for various interactions. As shown, there are three different caregivers 208 (caregiver #1 such as a physician, caregiver #2 such as a resident and caregiver #3 such as a nurse). Each caregiver 208 may spend different amounts of time and/or interact with the client context 202 a certain number of times e.g., at a certain frequency. These interactions may be represented statistically, per interaction type and per caregiver 208, as depicted by Figure 5. As shown, the frequency of interaction 'A' such as examining (i.e., 'interaction data') blood pressure data (i.e., 'client data') may vary by caregiver 208. The same applies for interaction 'B' such as examining (i.e., 'interaction data') infused vasoactive substances (i.e., 'client data'), interaction 'C' such as examining medication administration and interaction 'D' such as examining end-tidal carbon dioxide measurements. As depicted, it is possible to tell how frequently the client data (as part of the client context 202) was interacted with by each caregiver 208, which may be indicative of useful information for the user 216. For example, if a certain caregiver 208 did not spend much time on a certain task or spent a lot of time on a certain task, this may indicate something clinically useful for the user 216.

In some cases, the statistic may be indicative of frequency of certain actions. For example, the statistic may be indicative of at least one of: the frequency of certain actions taking place on a client monitor such as 'zoom-in' for certain waveforms, 'zoom-out' to look at the trend, adding new parameter to the screen and/or a pattern of changing alarm settings (e.g., per caregiver 208 and/or per client 204). This might be useful for various purposes such as assisting in determining client condition, providing guidance on a client 204 in a critical state, etc.

In some cases, the statistic may be indicative of waveforms on a client monitor that are observed together (at the same time), e.g., per caregiver 208 and/or per client 204.

In some cases, the statistic may be indicative of waveforms and quantified parameters (e.g., client data such as vital signs) on a client monitor observed together e.g., per caregiver 208 and/or per client 204.

In some cases, the statistic may be indicative of alerts/alarms ignored or accepted and/or actions taken upon each accepted alert/alarm e.g., per caregiver 208 and/or per client 204.

In some cases, the statistic may be indicative of recording the type and frequencies of interventions (e.g., administering medications, etc.) e.g., per caregiver 208 and/or per client 204.

In some cases, the statistic may be indicative of a degree of customization of a client monitor such as accounting for a combination of waveforms, thresholds, client diagnosis and prognosis, and/or caregiver 208 specialization e.g., per caregiver 208 and/or per client 204.

In some cases, the statistic may be indicative of changes in caregiver 208 determined thresholds (e.g., magnitude, coefficient of variation) e.g., per caregiver 208 and/or per client 204. In some cases, a threshold may be set higher or lower than a default threshold, which may be indicative of a pattern associated with a certain caregiver 208 or client 204.

In some cases, the statistic may be indicative of a divergence from SOPs. For example, a complex case may follow a singular trial and error process, which be apparent from e.g., multiple diagnostic tests being performed, disparate or unusual set of tests being performed, etc., with respect to the presently known client profile.

In some cases, the statistic may be indicative of how many additional monitors/medical equipment are used for a certain client such as syringes, ventilators, electroencephalography (EEG) leads, etc. In other words, if the equipment used is more or less 'invasive' than usual for the presently known client profile, this may be indicative of a potential issue such as unpredicted condition for the client 204 or excessive treatment given the client's 204 last known condition.

In some cases, the statistic may depict a pattern that may indicate whether or not there is a deviation from an expectation. This pattern may refer to something about the client 204 and/or the caregiver 208. In the case of the client 204, this pattern may highlight a potential issue in terms of their care plan or an unexpected problem. In the case of the caregiver 208, this pattern may highlight a potential issue with the care provided, how the caregiver 208 interacts with the client context 202 and/or be used as training to help other caregivers 208 understand how to improve their interactions with the client context 202. For example, statistic can be used to generate guidance to treat critical patients. In another example, statistic can be used to estimate the protocol adherence, which may be of interest to administrators.

Figure 6 refers to a method 600 of using an interaction statistic according to an embodiment. Reference is made to Figure 2 in the description of the method 600. The method 600 may be implemented by the computing system 212 and may be used to generate statistics such as described above. Certain blocks of the method 600 may be omitted for some embodiments, or implemented in a different order to that shown by Figure 6.

In some embodiments, the method 600 comprises, at block 602, using a statistical model to generate at least one interaction statistic based on the interaction data and/or at least one detected pattern in the interaction data.

The method 600 further comprises, at block 604, generating the information based on the at least one interaction statistic.

The statistical model may define which quantities (e.g., 'metrics' such as described above) are to be extracted from the interaction data in order to generate the at least one interaction statistic. For example, the statistical model may be configured to extract the quantities of interest from the interaction data (e.g., within the stored metadata) based on what is requested by the user 216. The request may define at least one parameter such as a period of time of interest, which caregivers 208 to focus on, etc. The statistical model may extract the quantities of interest based on the at least one parameter. The statistical model may be further configured to calculate the statistic from the quantities. For example, if the metric is represented by a number of data points in a list and the statistic to be calculated is a comparison of different caregivers 208, the metric for each caregiver 208 may be collated and provided in a suitable format in the generated information (e.g., a cumulative number of interactions within a timeframe, percentage comparison of number, type, quality, etc. of interactions per caregiver (e.g., as shown by Figure 5), or whatever format is most appropriate for the requested information).

Thus, the statistical model may evaluate available quantities/metrics and provide this information in a statistical way via the generated information to facilitate analysis by the user 216. In some cases, the output of the statistical model may be a set of values which can be interpreted by the user's 216 computing device 214 and provided in a user-friendly form (e.g., a graph, map, list of numbers, etc.) as defined by an application of the computing device 214. In some cases, the output of the statistical model may be a file in the user-friendly form.

As highlighted above, it may be possible to determine the client's 204 condition based on the statistic.

As such, the statistic (e.g. any number and/or combination of the statistics listed above) can be used as an additional information together with the client data to determine client condition. This can be enabled using a model (e.g., based on a set of rules/implemented with a database) of different interaction statistic(s) associated with the corresponding client condition. Such a model can be built based on the information collected for a variety of client conditions and several caregivers 208, together with the client data. An example of statistical measurements to determine the client profile is the frequency of measurements (e.g., as performed as part of the interactions with the client context 202). For example, unstable encounters may be more likely to be associated with a higher number of measurements (>15/day) than stable encounters. The frequency of measurements may provide a more granular overview per vital signs type. The status of the client 204 can be derived by measurement type and/or frequency. Such statistics may quantity a 'gut feeling' of the caregivers 208 involved with the care of a client 204, whether or not any of the caregivers 208 are aware of this gut feeling. For example, an individual caregiver 208 interaction may not provide a good indication of the client condition. However, if multiple interactions are considered with respect to multiple caregivers 208, this may be a better representation of the 'gut feeling' about the client condition. In some cases, this 'gut feeling' can even detect deterioration of the client 204 earlier than any algorithm because unlike the algorithms, the caregivers 208 see patterns in all aspects of the client 204. It can be considered that this 'gut feeling' is a particularly useful element in the diagnostic process of an experienced caregiver 208. Here, the underlying premise is that the caregiver 208 sees something that is troubling them and they react by increasing the 'analytical reasoning' in the diagnostic process, resulting in an increase inflow of information from the client 204 (e.g. measurement frequency).

In some cases, the computing system 212 may use the interaction statistic to determine the client condition, which can include at least one of: the time the caregiver 208 is looking at data from the client 204 (e.g., which can also refer to any interruptions that occur when looking at the data), the amount of data flowing in from the client 204 (e.g., measurement frequency, etc.) and/or the type of data that is coming from the client 204 (e.g., complex measurements, expensive measurements), etc.

It is common for clinicians (e.g., radiologists) to be disturbed/interrupted during their data analyses, for example by phone calls asking for their advice. The same is also true to physicians and nurses.

Having records of when the disturbance happens can be also included in the interaction data that is collected. Knowing the activity (e.g. data being analyzed/visualized, computation performed, setting adapted, etc.) of the caregiver 208 during interruption can also be useful. Such information may be used to weight (even ignore, reset, repeat) all interactions prior to interruption, restart the interactions from the right point and/or generate a summary of interactions specific for the current user and the interrupted session. The duration and nature of the interruption can be also taken into account while deciding from where to restart the analysis. Thus, the interaction data and/or generation information may provide an indication of any interruptions and/or whether that interruption has any impact on the understanding of the interaction between the caregiver 208 and the client context 202.

A change of interaction statistic may be dependent on the experience of the caregiver 208. For example, an increase in the interaction statistic of an experienced caregiver 208 might indicate that the client condition is unstable and/or the diagnosis is not accurate/clear.

In some embodiments, the method 600 comprises, at block 606, using a status determining model to determine a potential status (e.g., condition, change in status/condition, etc.) of the client 204 based on the interaction statistic. The status determining model may be implemented by or accessible to the computing system 212. The status determining model may take, as its input, the interaction statistic and evaluate a potential issue or flag to raise based on this statistic. In some cases, the status determining model may be configured with a predefined threshold which, if crossed by the input interaction statistic, indicates a change in status of the client 204. In some cases, the status determining model may learn about an interaction pattern associated with a client 204 based on the interaction statistics from a specified time period and then, if that pattern changes, the status determining model may flag a potential change. In some cases, the status determining model may be configured to output a prediction of what the interaction statistic means e.g., based on predefined rules which associate a certain status with a certain statistic/pattern, etc.

In some embodiments, the status determining model is configured to determine the potential status based on a client profile associated with the client 204 and/or based on an analysis of the at least one interaction between the at least one caregiver 208 and the client context 202.

In some embodiments, the generated information may be used to improve collaboration and/or workflow efficiency. Such embodiments may be based on the generated metric and/or statistic and may provide information to the user 216.

For example, the generated information may be used to improve the collaboration (e.g., consultation, teleconsultation) and workflow (e.g., shift handover) within a caregiving team. The teleconsultation between the nurse and the intensivists can sometimes be not effective and/or misleading. While having the teleconsultation, if the caregiver 208/user 216 gets an interaction statistic of the nurse with the client monitor, they can get a chance to reduce the bias of the nurses' description of the client 204. Showing this interaction statistic at the time of shift handover can also improve the completeness of the handover process and/or reduce staff dissatisfaction. The monitor can store the interactions between the medical staff members (e.g., linked to a certain ID) and a certain client 204, so that, during a shift the chronology of events can be traced back and reported. The nurse can recall this chronology during the handover to facilitate the process and increase accuracy.

Figure 7 refers to a method 700 of providing information to a user according to an embodiment. Reference is made to Figure 2 in the description of the method 700. The method 700 may be implemented by the computing system 212 and may be used to generate statistics such as described above. Certain blocks of the method 700 may be omitted for some embodiments.

The method 700 comprises, at block 702, identifying a point in a workflow (e.g., indicating interactions that occur as part of providing care) considered to be relevant to a care plan (e.g., tailored to the client 204). The workflow is associated with providing care to the client 204 as part of the care plan. Examples of points to be identified include 'handover', formal rounds or any other point in the workflow where information may be usefully shared. Such identified points may be predefined or automatically detected.

The method 700 further comprises, at block 704, providing the information (i.e., the 'generated information') to the user at the identified point.

In some embodiments, the generated information may be used to improve management/monitoring of use of alerts/alarms. In some cases, a statistic representative of action taken with respect to certain alerts may be used together with a statistic representative of interventions to prioritize or deprioritize certain alerts/alarms.

In an example scenario, the client 204 may be given a diuretic medicine to reduce the fluid build-up due to a congestive heart failure condition. It is common that diuretics will lower the blood pressure by flushing salt out of the body. Now the client monitors will in general not know why the blood pressure is going down and the default alarm might trigger. An intelligent client monitor (i.e., 'equipment 206') with the help of the contextual information (e.g., the interaction data showing that diuretic medicine was administered) can access the interaction statistic to check how often the alarms are ignored/attended when a specific intervention has been made. This can lead to a new alarm management system where the alarms caused by obvious reasons (i.e. known to the caregivers 208) are masked or deprioritized. Additionally, the alarms that are attended can be put a higher priority to improve the clinical outcome and quality of care. A reduction in false alarms and/or an increase in non-obvious alarms may also increase the trust of the caregiver 208 on the alarm management systems.

In another example scenario, a client 204 such as an ICU patient may suffer from Obstructive Sleep Apnea (OSA). The alarm system may trigger a 'blue' alarm any time SPO₂ falls below a certain level and attention needs to be given to the patient for this particular reason. A 'red' alarm may be triggered any time SPO₂ goes below a critical level and medical staff attention and intervention is required at the bedside. However, apneas and consequent desaturations are usual in OSA patient. They are chronic conditions and do not expose, per se, the patient to a critical situation. These alarms, however, may require time, action, extra-attention of the medical staff. The intelligent patient monitors as described above may evaluate these specific cases and check the patient history, the medical staff interaction with the alarm system and can notify OSA events in a different way (e.g. notification of the central station and monitor), deescalating the severity of the alarm itself, because of the lower risk associated to that event in that specific patient, such as apnea and desaturation in OSA patient.

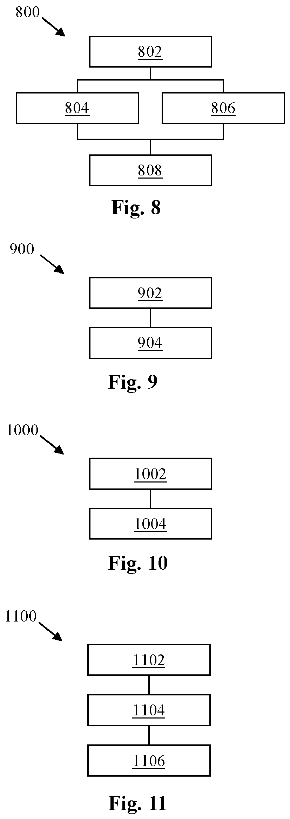

Figure 8 refers to a method 800 of determining a priority level according to an embodiment. Reference is made to Figure 2 in the description of the method 800. The method 800 may be implemented by the computing system 212 and may be used to determine a priority level of an alert/alarm such as described above. Certain blocks of the method 800 may be omitted for some embodiments, or implemented in a different order to that shown by Figure 8.

In response to receiving an alert associated with the client context 202, the method 800 comprises, at block 802, using a priority determining model to determine a priority level for the alert based on the interaction data. The priority determining model may be pre-configured or otherwise configured to identify patterns such as an association between a certain interaction and an alert. Rules may be specified by the priority determining model to indicate whether an alert can be linked to a known interaction and whether or not that alert is anticipated as a result of the interaction. If the alert is expected based on the interaction (and the cause of the alert is acceptable in this context), the alert may be de-prioritized or have a lower priority. Similarly, if the alert is not expected based on the interaction (and the cause of the alert is not acceptable in this context), the alert may have a higher priority.

Thus, in response to the interaction data being indicative of an expected cause of the alert that is not an event of concern, the method 800 further comprises, at block 804, determining that the priority level is lower than if the alert was received in the absence of the interaction data

However, in response to the interaction data being indicative of an event of concern, the method 800 further comprises, at block 806, determining that the priority level is higher than if the alert was received in the absence of the interaction data.

The method 800 further comprises, at block 808, indicating the determined priority level with the generated information. The rules for operating the 'intelligent' monitor (of the equipment 206) may be reviewed and/or updated based on the generated information such as the statistic.

In some embodiments, privacy issues may be avoided or reduced if each caregiver 208 defines which interaction data is to be made available. Thus, the statistic may depend on what a caregiver 208 defines what interaction data can be submitted, and hence used, for generating the statistic. For example, a caregiver 208 may find it acceptable to share everything they have done with the client data. In another example, a caregiver 208 may be willing to share certain data, but not anything else (such as, for how long they were visualized, for what simulation they were used, which parts of the data were analyzed (zoomed), etc.).

Figure 9 refers to a method 900 of determining whether information is to be unavailable according to an embodiment. Reference is made to Figure 2 in the description of the method 900. The method 900 may be implemented by the computing system 212 and may be used to avoid or reduce privacy issues such as described above. Certain blocks of the method 900 may be omitted for some embodiments.

The method 900 comprises, at block 902, determining whether the caregiver 208 has specified (e.g., via an input to the equipment 206, their device 210 or previously entered) whether at least part of the information (e.g., associated with at least part of at least one of their interactions) is to be unavailable to the user 216. This could refer to any user 216 or a certain user 216 or set of users 216.

In response to determining that at least part of the information is to be unavailable to the user, the method 900 comprises, at block 904, preventing the specified part of the information from being made available via the generated information. This can be achieved in various ways. In some cases, the computing system 212 may decide not to store the information indicated as being supposed to be unavailable. In some cases, the computing system 212 may store the information but tag it in some way (e.g., store in a certain allocated storage, associate a tag with it, encrypt it, tokenize it or otherwise mask the information) so that any request for such information is denied by the computing system 212.

In some embodiments, the generated information may be used for loading a preferred interface setting associated with the equipment 206 and/or a device associated with the caregiver 208. This may be implemented as an automated system that loads the preferred user interface/user experience (UI/UX) (e.g., alarm settings, workflow, data view) settings of the equipment 208/device for a known caregiver 208 e.g., based on individual authentication.

In some embodiments, the caregiver 208 may store their (fixed) preferences manually (e.g., in or accessible to the computing system 212) and these preferences may be loaded or predefined UI/UX that are available per caregiver role. Thus, in some cases, the UI/UX that is loaded may correspond to the role of the caregiver 208.

In some embodiments, the computing system 212 learns the caregiver's 208 preferences through monitoring their interactions and/or analyzing their associated interaction data. For example, the computing system 212 may learn that caregivers 208 with the role of physiotherapist often watch the screen depicting the client's activity (e.g. measured by an accelerometer in the equipment 206) over time and often switch between that screen and the screen that shows the patient's SpO2 level. Although on the initial UX/UI of the equipment 206, the SpO2 level is shown on the home screen, the caregiver 208 needs to select from a menu and click through even more menus to arrive at the screen that shows the client's activity. After the computing system 212 has learned that physiotherapists often look at the patient's activity and switch between patient activity and SpO2 level, the computing system 212 may instruct the equipment 206 to show activity level and SpO2 level superimposed at the start screen as soon as a physiotherapist logs on to the system. Alternatively or additionally, a shortcut button could be made on the home screen that highlights this non-default view to the attention of the caregiver 208 based on their caregiver profile (e.g. their role).

Figure 10 refers to a method 1000 of determining a preference according to an embodiment. Reference is made to Figure 2 in the description of the method 1000. The method 1000 may be implemented by the computing system 212 and may be used to implement the caregiver's 208 preference in a device (such as described above). Certain blocks of the method 1000 may be omitted for some embodiments.

Thus, in some embodiments, the client context 202 comprises a client device (e.g., equipment 206) associated with the client 204. The client device comprises a user interface (e.g., a graphical user interface, audio user interface, etc.) with which the caregiver 208 interacts as part of the interaction.

In this regard, the method 1000 comprises, at block 1002, using the interaction data and/or the information to determine a preference of the caregiver 208 for their interaction with the client device. As already mentioned, the preference may be determined based on fixed choices and/or automatically learned by the computing system 212.

In response to determining that the user interface does not meet the preference, the method 1000 comprises, at block 1004, causing the user interface to be modified to meet the preference.

In some embodiments, the generated information may facilitate protocol management such as medication management and/or treatment guidance. For example, the recorded interaction(s) related to drug delivery or any other interventions may facilitate such protocol management. Such embodiments may enable displaying/showing this information at different displays to bring the interaction under the notice of the same/new caregiver 208 to avoid mistake.

Many clinical errors may be linked to (incorrect) administering of medications. A way to reduce medication errors involves introducing redundancy and additional, double, and sometime triple checks at different stages of medication prescription and administration. However, knowledge of different caregivers 208 is significantly different when it comes to medication. These differences also indicate a need for checks by different expertise levels.

There are certain types of medications such as high-alert medications that need additional attention because their harm can be significant on the client 204. Therefore, at least double checks may be recommended for such medications. The generated information described herein may be useful for such a purpose. For instance, the availability of the medicine in the list of medications that the patient can receive, or the permission to administer the medicine, can be only possible when the medicine has been verified by more than two clinicians (and this may be indicated within the interaction data). Additionally or alternatively, by including the information about how various caregivers 208 reviewed, approved, or in general used and processed the medications in the proposed overview, the generated information may be used by other caregivers 208 to work in a more efficient manner, because all the critical information will be readily available, and they will have the assurance of the required checks being performed.

In some embodiments, the generated statistic may be compared with a computer-interpretable SOP e.g., to allow the possibility of automatically estimating the protocol adherence. Additionally, all the missing steps can also be manually evaluated to measure their effect on the outcome.

Figure 11 refers to a method 1100 of using interaction data according to an embodiment. Reference is made to Figure 2 in the description of the method 1100. The method 1100 may be implemented by the computing system 212 and may be used to identify a deviation from a care protocol and/or a level of protocol adherence (such as described above). Certain blocks of the method 1100 may be omitted for some embodiments.

The method 1100 comprises, at block 1102, accessing a care protocol (e.g., defined by the team, hospital, government organization or other institution for a particular condition or set of conditions).

The method 1100 further comprises, at block 1104, identifying, based on the interaction data, a deviation from the care protocol and/or a measure of adherence to the care protocol.

In response to identifying the deviation and/or the measure of adherence, the method 1100 further comprises, at block 1106, indicating the deviation and/or the measure of adherence via the (generated) information.

In some embodiments, client care can may be made leaner (e.g., more efficient) by being more aware of the past interactions. This awareness may be provided based on the interaction data as well as the client data, to make client care leaner and/or reduce potential waste embedded in certain care processes. For example, during a hospital stay of a client 204, the caregivers 208 may have conducted multiple care activities (i.e., 'interactions'). Not all of these activities may be equally important. Therefore, an importance indicator may be attributed to all patient-clinician interaction data. In some cases, this could be done by correlating the interaction data with the vital signs and/or lab data, i.e., by evaluating the impact of certain activities on client condition. Actions and interactions critical for patient outcome can be highlighted, whereas less relevant ones may be candidates for further investigation to make certain processes leaner. In other words, an analysis of past interactions may be used to infer a potential relationship between certain parts of the interaction data and a health status/condition of the client 204. For example, where such a relationship exists, the interaction data that is relevant to an outcome for the client 204 may be inferred and highlighted as part of the generated information.

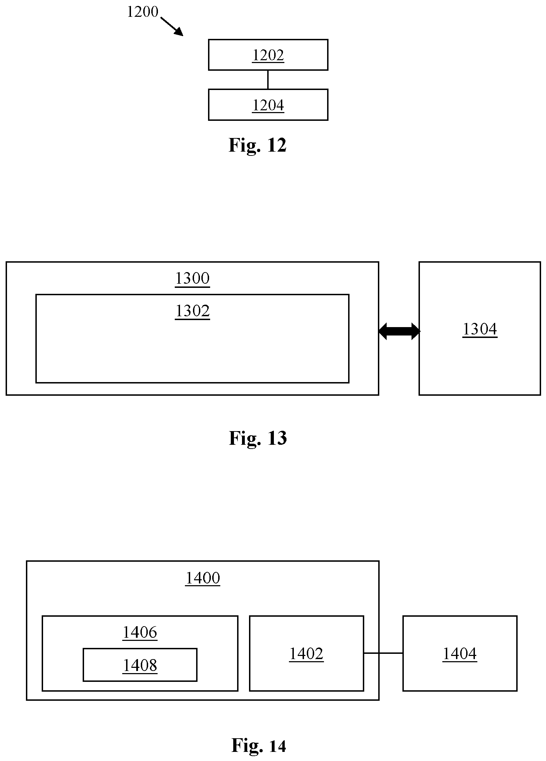

Figure 12 refers to a method 1200 of using a relationship identifying model according to an embodiment. Reference is made to Figure 2 in the description of the method 1200. The method 1200 may be implemented by the computing system 212 and may be used to infer a potential relationship (such as described above). Certain blocks of the method 1200 may be omitted for some embodiments.

The method 1200 comprises, at block 1202, using a relationship identifying model to identify a correlation between at least part of the interaction data and a measurement of a health status of the client 204 (e.g., a vital sign, etc.) that is indicative of an existence of a relationship between the part of the interaction data and the measurement of the health status. The relationship identifying model may be configured to identify whether there is a causal connection between the measurement and the interaction. For example, an interaction may be problematic and this may correlate to a certain measurement of the health status. In some cases, the relationship identifying model may implement predefined rules for identifying the correlation. In some cases, the relationship identifying model may learn such rules based on a monitored set of measurements and interaction data. In some cases, the relationship identifying model may associate certain measurements with certain interactions based on a temporal relationship. For example, proximity of time between measurements and interactions may indicate a high probability of there being a correlation whereas a significant amount of time between measurements and interactions may indicate a low probability of there being a correlation.

The relationship identifying model, as part of block 1202, further determines whether the part of the interaction data associated with the identified correlation is relevant to an outcome (e.g., a health outcome) for the client 204 based on a client profile associated with the client 204. For example, the correlation may or may not be relevant to the outcome. For example, if the correlation is relevant to the outcome, this could be positive or negative to the client 204.

In response to determining that the part of the interaction data is relevant to the outcome, the method 1200 further comprises, at block 1204, indicating such relevance via the information. Thus, if the correlation is relevant to the outcome, the indication of the relevance may be provided to the user 216 in response to the request, which may ensure that the correlation is investigated.

In some embodiments, a link to an existing protocol may be established based on a classification of the client 204. This may refer to a knowledge-based model of diagnostic reasoning where such reasoning may take various forms. For example, there may be two types of reasoning: analytical (e.g., medical protocol-based decision making such as based on a flowchart) and non-analytical reasoning (e.g., 'gut feeling'). Problem solving may be another example type of reasoning that includes both analytical and non-analytical components. Signs and symptoms may inform analytical reasoning whereas contextual information may inform non-analytical reasoning. The problem solving may lead to a 'most probable' diagnosis, prognosis and indicate an intervention to be carried out, if any. The information generated according to various embodiments described herein may support such a knowledge-based model by providing additional input, including one or both of analytical and non-analytical components.

In some cases, it may be possible to compute the interaction statistic and some client similarity model/measure (e.g., based on previous similar clients) based on client's physiological information (e.g., as represented by the client data). The existing client similarity may be used together with the interactions statistic to classify a new client 204 as critical or non-critical. Once the patient is classified, an interaction rule set/database (of the computing system 212) can be queried to retrieve the list of interactions corresponding to a particular client condition. These retrieved interactions can then be used as a guidance for treating the new client 204. This guidance can relate to any of the interactions e.g., intervention, treatment, management, etc.

By way of example, new-onset Atrial Fibrillation (AF) can be triggered by accelerated atrial remodeling and arrhythmogenic triggers encountered during critical illness. The new-onset AF may often lead to decreased cardiac output and hemodynamic compromise. Thus, new-onset AF is both a marker of disease severity as well as a likely contributor to poor outcomes, similar to other manifestations of organ dysfunction during critical illness. Acute management priorities in new-onset AF during critical illness may have an associated protocol for identification of new-onset AF during critical illness. Such a protocol may involve (1) assessment for hemodynamic compromise requiring urgent DCCV (direct current cardioversion), (2) removal of potential offending agents, (3) reversal of inciting acute factors, and (4) treatment to reduce rate or convert to sinus if AF persists and is associated with adverse effects.

Needless to say that to detect the new onset of AF, an Electrocardiogram (ECG) will be reviewed with an utmost importance. Additionally, depending on the source (structural remodeling/electrical remodeling) of AF, caregivers 208 may be interested in reviewing different physiological parameters.

On the other hand, if the intervention is recorded then, from the intervention, the probable diagnosis can be interpreted. After the onset of AF, if beta-agonists are withdrawn then the suspected cause would be pulmonary induced electrical remodeling.

Thus, by following a protocol based on a similarity model and monitoring the interactions/client data, this may point to certain diagnosis, treatment, etc., and indicate whether or not the care plan being followed is the most appropriate one given the latest available information.

Figure 13 is a schematic drawing of a non-transitory machine-readable medium 1300 for implementing various embodiments described herein. The machine-readable medium 1300 stores instructions 1302 which, when executed by processing circuitry 1304, cause the processing circuitry 1304 to implement the method of any of the embodiments described herein. The machine-readable medium 1300 and/or the processing circuitry 1304 may be implemented by the computing system 212 of Figure 2.

Figure 14 is a schematic drawing of apparatus 1400 for implementing various embodiments described herein. The apparatus 1400 may be implemented by the computing system 212 of Figure 2.

The apparatus 1400 comprises processing circuitry 1402. The processing circuitry 1402 is configured to communicate with an interface 1404. The interface 1404 may be any interface (wireless or wired) implementing a communications protocol to facilitate exchange of data with other devices such as the equipment 206, data-gathering device 210 and/or user devices 214.

The apparatus 1400 further comprises a machine-readable medium 1406 (e.g., non-transitory or otherwise) storing instructions 1408 which, when executed by the processing circuitry 1402, cause the processing circuitry 1402 to implement various embodiments described herein (e.g., method 100 or any of the associated embodiments).

Methods, machine-readable media and apparatus described herein may be implemented at the computing system 212. However, other devices described in the system 200 may assist with the implementation of such methods, machine-readable media and apparatus (e.g., due to sending/receiving data to/from the computing system 212).

While the invention has been illustrated and described in detail in the drawings and foregoing description, such illustration and description are to be considered illustrative or exemplary and not restrictive; the invention is not limited to the disclosed embodiments.

One or more features described in one embodiment may be combined with or replace features described in another embodiment.

Embodiments in the present disclosure can be provided as methods, systems or as a combination of machine-readable instructions and processing circuitry. Such machine-readable instructions may be included on a non-transitory machine (for example, computer) readable storage medium (including but not limited to disc storage, CD-ROM, optical storage, flash storage, etc.) having computer readable program codes therein or thereon.

The present disclosure is described with reference to flow charts and block diagrams of the method, devices, and systems according to embodiments of the present disclosure. Although the flow charts described above show a specific order of execution, the order of execution may differ from that which is depicted. Blocks described in relation to one flow chart may be combined with those of another flow chart. It shall be understood that each block in the flow charts and/or block diagrams, as well as combinations of the blocks in the flow charts and/or block diagrams can be realized by machine readable instructions.

The machine-readable instructions may, for example, be executed by a general-purpose computer, a special purpose computer, an embedded processor, or processors of other programmable data processing devices to realize the functions described in the description and diagrams. In particular, a processor or processing circuitry, or a module thereof, may execute the machine-readable instructions. Thus, functional modules of apparatus and other devices described herein may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate array etc. The methods and functional modules may all be performed by a single processor or divided amongst several processors.

Such machine-readable instructions may also be stored in a computer readable storage that can guide the computer or other programmable data processing devices to operate in a specific mode.

Such machine-readable instructions may also be loaded onto a computer or other programmable data processing devices, so that the computer or other programmable data processing devices perform a series of operations to produce computer-implemented processing, thus the instructions executed on the computer or other programmable devices realize functions specified by block(s) in the flow charts and/or in the block diagrams.

Further, the teachings herein may be implemented in the form of a computer program product, the computer program product being stored in a storage medium and comprising a plurality of instructions for making a computer device implement the methods recited in the embodiments of the present disclosure.

Elements or steps described in relation to one embodiment may be combined with or replaced by elements or steps described in relation to another embodiment. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (100), comprising:
receiving (102) a request associated with a user for information about a client; and
in response to the request, generating (104) information indicative of at least one interaction between at least one caregiver and a client context associated with the client based on interaction data obtained responsive to the at least one interaction, wherein the information is customized to the user based on a user profile associated with the user.

2. The method of claim 1, wherein the interaction data is indicative of at least one of: a behavior of the caregiver as part of the at least one interaction; an activity of the caregiver as part of the at least one interaction; a response of the caregiver to the client context as part of the at least one interaction; a location of the caregiver as part of the at least one interaction; a time of the at least one interaction; equipment used by the caregiver as part of the at least one interaction; at least one type and/or setting of the equipment used by the caregiver as part of the at least one interaction.

3. The method of any of claims 1 to 2, wherein:
the generated information is indicative of a caregiver profile associated with the caregiver; and
the caregiver profile comprises at least one of:
a role of the caregiver;
experience of the caregiver;
historical data about the caregiver's interactions with: other clients; at least one other caregiver and/or the user; and/or
a performance characteristic of the caregiver.

4. The method of any of claims 1 to 3, wherein generating the information comprises generating an interaction metric for quantifying the caregiver's understanding of a status of the client based on a predefined rule specifying how to generate the interaction metric based on at least one criterion associated with the at least one interaction.

5. The method (600) of any of claims 1 to 4, comprising:
using (602) a statistical model to generate at least one interaction statistic based on the interaction data and/or at least one detected pattern in the interaction data; and
generating (604) the information based on the at least one interaction statistic.

6. The method (600) of claim 5, comprising:
using (606) a status determining model to determine a potential status of the client based on the interaction statistic.

7. The method of claim 6, wherein the status determining model is configured to determine the potential status based on a client profile associated with the client and/or based on an analysis of the at least one interaction between the at least one caregiver and the client context.

8. The method (700) of any of claims 1 to 7, comprising:
identifying (702) a point in a workflow considered to be relevant to a care plan, wherein the workflow is associated with providing care to the client as part of the care plan; and
providing (704) the information to the user at the identified point.

9. The method (800) of any of claims 1 to 8, comprising:
in response to receiving an alert associated with the client context, using (802) a priority determining model to determine a priority level for the alert based on the interaction data, wherein:
in response to the interaction data being indicative of an expected cause of the alert that is not an event of concern, determining (804) that the priority level is lower than if the alert was received in the absence of the interaction data; or
in response to the interaction data being indicative of an event of concern, determining (806) that the priority level is higher than if the alert was received in the absence of the interaction data; and
indicating (808) the determined priority level with the generated information.

10. The method (900) of any of claims 1 to 9, comprising:
determining (902) whether the caregiver has specified whether at least part of the information is to be unavailable to the user; and
in response to determining that at least part of the information is to be unavailable to the user, preventing (904) the specified part of the information from being made available via the generated information.

11. The method (1000) of any of claims 1 to 10, wherein the client context comprises a client device associated with the client, wherein the client device comprises a user interface with which the caregiver interacts as part of the interaction, and wherein the method comprises:
using (1002) the interaction data and/or the information to determine a preference of the caregiver for their interaction with the client device; and
in response to determining that the user interface does not meet the preference, causing (1004) the user interface to be modified to meet the preference.

12. The method (1100) of any of claims 1 to 11, comprising:
accessing (1102) a care protocol;
identifying (1104), based on the interaction data, a deviation from the care protocol and/or a measure of adherence to the care protocol; and
in response to identifying the deviation and/or the measure of adherence, indicating (1106) the deviation and/or the measure of adherence via the information.

13. The method (1200) of any of claims 1 to 12, comprising:
using (1202) a relationship identifying model to:
identify a correlation between at least part of the interaction data and a measurement of a health status of the client that is indicative of an existence of a relationship between the part of the interaction data and the measurement of the health status; and
determine whether the part of the interaction data associated with the identified correlation is relevant to an outcome for the client based on a client profile associated with the client; and
in response to determining that the part of the interaction data is relevant to the outcome, indicating (1204) such relevance via the information.

14. A non-transitory machine-readable medium (1300) storing instructions (1302) which, when executed by processing circuitry (1304), cause the processing circuitry to implement the method of any of claims 1 to 13.

15. Apparatus (1400) comprising:
processing circuitry (1402) configured to communicate with an interface (1404) for receiving (102) a request associated with a user for information about a client; and
a machine-readable medium (1406) storing instructions (1408) which, when executed by the processing circuitry, cause the processing circuitry to, in response to the request, generate (104) information indicative of at least one interaction between at least one caregiver and a client context associated with the client based on interaction data obtained responsive to the at least one interaction, wherein the information is customized to the user based on a user profile associated with the user.
